# EUROPEAN PATENT SPECIFICATION

(11) **EP 2 785 335 B1**
(45) Date of publication and mention of the grant of the patent: **14.06.2017**
(21) Application number: 12791780.5
(22) Date of filing: 29.11.2012
(51) Int. Cl.: A61K 31/192, A61K 31/445, A61P 17/00, A61P 17/12

(54) **METHODS AND PHARMACEUTICAL COMPOSITIONS FOR THE TREATMENT OF DARIER DISEASE**
VERFAHREN UND PHARMAZEUTISCHE ZUSAMMENSETZUNGEN ZUR BEHANDLUNG VON MORBUS DARIER
PROCÉDÉS ET COMPOSITIONS PHARMACEUTIQUES POUR LE TRAITEMENT DE LA MALADIE DE DARIER

(30) Priority: 29.11.2011 EP 11306578
(43) Date of publication of application: 08.10.2014
(73) Proprietor: Institut National de la Santé et de la Recherche Médicale (INSERM), 75013 Paris (FR); Université Paris Descartes, 75006 Paris (FR)
(72) Inventor: HOVNANIAN, Alain, F-75743 Paris Cedex 15 (FR); SAVIGNAC, Magali, F-31024 Toulouse Cedex 3 (FR)
(74) Representative: Nony
(86) International application number: PCT/EP2012/073936
(87) International publication number: WO 2013/079580

(56) References cited:
- WO-A1-2010/102988
- US-A1- 2010 130 603
- COOPER SUSAN M ET AL: "Darier's disease: epidemiology, pathophysiology, and management.", AMERICAN JOURNAL OF CLINICAL DERMATOLOGY 2003, vol. 4, no. 2, 2003, pages 97-105, XP009166773, ISSN: 1175-0561
- KREBS M P ET AL: "Quality control of integral membrane proteins", TRENDS IN BIOCHEMICAL SCIENCES, ELSEVIER, HAYWARDS, GB, vol. 29, no. 12, 1 December 2004 (2004-12-01), pages 648-655, XP004641483, ISSN: 0968-0004, DOI: 10.1016/J.TIBS.2004.10.009
- LOO TIP W ET AL: "Chemical and pharmacological chaperones as new therapeutic agents", EXPERT REVIEWS IN MOLECULAR MEDICINE, CAMBRIDGE UNIVERSITY PRESS, CAMBRIDGE, vol. 9, no. 16, 1 June 2007 (2007-06-01), pages 1-18, XP009147295, ISSN: 1462-3994, DOI: 10.1017/S1462399407000361
- HOVNANIAN A: "Darier's disease: from dyskeratosis to endoplasmic reticulum calcium ATPase deficiency", BIOCHEMICAL AND BIOPHYSICAL RESEARCH COMMUNICATIONS, ACADEMIC PRESS INC. ORLANDO, FL, US, vol. 322, no. 4, 1 October 2004 (2004-10-01), pages 1237-1244, XP004536608, ISSN: 0006-291X, DOI: 10.1016/J.BBRC.2004.08.067
- MAGALI SAVIGNAC ET AL: "Darier disease : A disease model of impaired calcium homeostasis in the skin", BIOCHIMICA ET BIOPHYSICA ACTA. MOLECULAR CELL RESEARCH, ELSEVIER SCIENCE PUBLISHERS, AMSTERDAM, NL, vol. 1813, no. 5, 4 December 2010 (2010-12-04), pages 1111-1117, XP028190528, ISSN: 0167-4889, DOI: 10.1016/J.BBAMCR.2010.12.006 [retrieved on 2010-12-15]
- ONOZUKA T ET AL: "Possible role of endoplasmic reticulum stress in the pathogenesis of Darier's disease", JOURNAL OF DERMATOLOGICAL SCIENCE, ELSEVIER SCIENCE PUBLISHERS, SHANNON, IE, vol. 41, no. 3, 1 March 2006 (2006-03-01), pages 217-220, XP027954676, ISSN: 0923-1811 [retrieved on 2006-03-01]
- SAVIGNAC M ET AL: "Evidence of endoplasmic reticulum stress response upregulation in Darier keratinocytes which impairs E-cadherin trafficking, and reduces adherens junctions assembly", JOURNAL OF INVESTIGATIVE DERMATOLOGY, vol. 131, no. Suppl. 1, April 2011 (2011-04), page S67, XP002691676, & ANNUAL MEETING OF THE SOCIETY-FOR-INVESTIGATIVE-DERMATOLOGY; PHOENIX, AZ, USA; MAY 04 -07, 2011
- TAKAHASHI K ET AL: "Cannabinoid and vanilloid agonists as novel therapeutics for Darier's disease", JOURNAL OF INVESTIGATIVE DERMATOLOGY, NATURE PUBLISHING GROUP, GB, vol. 128, no. Suppl. 1, 17 May 2008 (2008-05-17), page S53, XP009158601, ISSN: 0022-202X
- BERNIER V ET AL: "Pharmacological chaperones: Potential treatment for conformational diseases", TRENDS IN ENDOCRINOLOGY AND METABOLISM 200407 US, vol. 15, no. 5, July 2004 (2004-07), pages 222-228, XP002691677, ISSN: 1043-2760
- PAPP E ET AL: "Chemical chaperones: mechanisms of action and potential use.", HANDBOOK OF EXPERIMENTAL PHARMACOLOGY 2006, no. 172, 2006, pages 405-416, XP009166859, ISSN: 0171-2004

## Description

### FIELD OF THE INVENTION:

The present invention relates to methods and pharmaceutical compositions for the treatment of Darier disease.

### BACKGROUND OF THE INVENTION:

Darier disease (DD) is a keratinization disorder characterized by the development of keratotic papules in seborrheic areas and specific nail anomalies. The prevalence is estimated at around 1/50,000. Onset of the disease usually occurs around puberty. Patients present with greasy and colored (yellow-brown or brown) keratotic papules, which may be isolated or grouped forming plaques. The skin lesions often become infected and malodorous, and are responsible for major discomfort. They may be exacerbated by exposure to sunlight or artificial UVB radiation, heat, sweating, friction, and infections. The sites of predilection are the seborrheic areas of the trunk and face: upper chest, back, sides of the neck, forehead, ears, and scalp. The flexures are also frequently involved (the groins, axillae, and anogenital region). Hands and feet may also show discrete papules on the dorsal surfaces. Careful examination of the palms and soles frequently reveals small pits or punctuated keratoses that are highly suggestive, if not specific, of DD. Nail abnormalities are almost constant and highly suggestive. The nails show the specific combination of red and white longitudinal stripes and present subungual hyperkeratosis. They are fragile and have a V-shaped defect. The hard palate, oral mucosa, esophagus, vulva and rectum may be the site of whitish small papules, often densely grouped (leukoplakia). Patients have an increased susceptibility to herpes simplex and pyogenic infections. Severity of the disease is highly variable, even within the same family.

DD is caused by mutations in the *ATP2A2* gene (12q23-q24.1) encoding a Ca²⁺ pump of the endoplasmic reticulum. *ATP2A2* encodes the sarco/endoplasmic reticulum Ca²⁺-ATPase isoform 2 (SERCA2), a calcium pump which transports Ca²⁺ from the cytosol to the lumen of endoplasmic reticulum (ER) to maintain a high Ca²⁺ concentration in this organelle. Functional studies of *ATP2A2* mutations identified in DD have shown that they lead to loss of Ca²⁺ transport of the pump. As a consequence of loss of SERCA2 Ca²⁺ transport, Darier keratinocytes (DK) display depleted ER Ca²⁺ store.

The diagnosis of DD is based on histological examination of skin lesion biopsies revealing hyperkeratosis, focal dyskeratosis and suprabasal acantholysis. Differential diagnoses include Hailey-Hailey disease, pemphigus and warty dyskeratoma (see these terms), as well as transient acantholytic dermatosis.

Management is symptomatic. Patients should avoid sun and heat. Emollients containing urea or lactic acid are of benefit for more limited lesions. Topical application of tretinoin or isotretinoin is effective against hyperkeratosis, but the risk of irritation limits their use. Topical steroids may reduce irritation, but they are not effective when used alone. Retinoids such as tazarotene are better tolerated. In case of severe disease, acitretin (an oral retinoid) is the most effective treatment, but possible side-effects must be monitored. Depression and neuropsychological manifestations have been reported and specific psychological support may be necessary. Accordingly, there is a need in the art to identify new pharmacologic targets for the treatment of DD.

### SUMMARY OF THE INVENTION:

The present description relates to methods and pharmaceutical compositions for the treatment of Darier disease. More particularly, the present description relates to an agent that is able to restore the E-cadherin trafficking in DK and the formation of adherens junctions for use in a method for treating patients with Darier disease. In particular, the present description relates to an agent selected from the group consisting of molecular, chemical and pharmacologic chaperones for use in a method for treating patients with Darier disease.

The present invention relates to an agent that is able to restore the E-cadherin trafficking in Darier keratinocytes and the formation of adherens junctions for use in treating a patient with Darier disease, wherein said agent is selected from the group consisting of chemical chaperones and pharmacologic chaperones, the chemical chaperone being 4-phenyl butyric acid (PBA), and the pharmacological chaperone being 1,5-(butylimino)-1,5-dideoxy-D-glucitol (miglustat).

### DETAILED DESCRIPTION OF THE INVENTION:

Darier disease is a severe dominant genetic skin disorder characterized by loss of cell-to-cell adhesion and abnormal keratinization. The inventors previously identified the defective gene, *ATP2A2,* which encodes SERCA2, a Ca²⁺-ATPase pump of the endoplasmic reticulum (ER). Here, they show that Darier keratinocytes (DK) display a constitutive ER stress response and immature adherens junctions (AJ) formation. These defects directly result from loss of SERCA2 function since treating normal keratinocytes with thapsigargin, a SERCA2 inhibitor, recapitulates ER stress and immature AJ. They demonstrate that abnormal AJ in DK is due to a selective defect in E-cadherin trafficking which is retained in the ER. Indeed, expression of constitutive E-cadherin to the plasma membrane restores catenin recruitment to the cell membrane. Treatment of DK with 4-phenylbutyric acid, a chemical chaperone that reduces ER stress, or with Miglustat a pharmacological chaperone, restores mature AJ formation, identifying these molecules as promising therapeutic agents in Darier patients.

Accordingly, the present invention relates to an agent that is able to restore the E-cadherin trafficking in DK and the formation of adherens junctions for use in a method for treating patients with Darier disease. In particular, the present invention relates to an agent selected from the group consisting of molecular, chemical and pharmacologic chaperones for use in a method for treating patients with Darier

The agent may be any type of chemical compound. The agent may be a small molecule, organometallic complex, an inorganic compound, a protein, a glycoprotein, a peptide, a carbohydrate, a lipid, or a nucleic acid. In certain embodiments, the agent is a small molecule.

Typically, the agent related to the invention is a chemical chaperone. A "chemical chaperone" has its general meaning in the art and refers to a compound known to stabilize protein conformation against denaturation (e.g., chemical denaturation, thermal denaturation), thereby preserving protein structure and function (Welch et al. Cell Stress Chaperones 1:109-115, 1996). In certain embodiments, the "chemical chaperone" is a small molecule or low molecular weight compound. Preferably, the "chemical chaperone" is not a protein.

Examples of chemical chaperones include but are not limited to glycerol, deuterated water (D2O), dimethylsulfoxide (DMSO), trimethylamine N-oxide (TMAO), glycine betaine (betaine), glycerolphosphocholine (GPC) (Burg et al. Am. J. Physiol. (Renal Physiol. 43):F762-F765, 1998), 4-phenyl butyrate or 4-phenyl butyric acid (PBA), methylamines, and tauroursodeoxycholic acid (TUDCA) or any derivative thereof.

In certain embodiments, the chemical chaperone according to the description is a derivative of 4-phenyl butyrate with the formula: 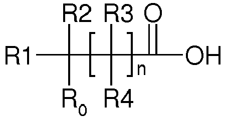
wherein n is 1 or 2;
R0 is aryl, heteroaryl, or phenoxy, wherein the aryl, heteroaryl, and phenoxy being unsubstituted or substituted with, independently, one or more halogen, hydroxy, or lower alkyl (C1-C6) groups;
R1 and R2 are independently H, lower alkoxy, hydroxy, lower alkyl or halogen; and
R3 and R4 are independently H, lower alkyl, lower alkoxy or halogen; or
a pharmaceutically-acceptable salt thereof; or a mixture thereof. In certain embodiments, R0 is a substituted or unsubstituted phenyl ring. In certain embodiments, R0 is an unsubstituted phenyl ring. In other embodiments, R0 is a monosubstituted phenyl ring. In yet other embodiments, R0 is a disubstituted phenyl ring. In still other embodiments, R0 is a trisubstituted phenyl ring. In certain embodiments, R0 is a phenyl ring substituted with 1, 2, 3, or 4 halogen atoms. In certain embodiments, R0 is a substituted or unsubstituted heteroaryl ring. In certain embodiments, R0 is a naphthyl ring. In certain embodiments, R0 is five- or six-membered, preferably six-membered. In certain embodiments, R1 and R2 are both hydrogen. In certain embodiments, n is 1. In other embodiments, n is 2. In certain embodiments, all R3 and R4 are hydrogen. In other embodiments, at least one R3 or R4 is hydrogen. In certain embodiments, the compound is used in a salt form (e.g., sodium salt, potassium salt, magnesium salt, ammonium salt, etc.) Other derivatives useful in the present description are described in U.S. Pat. No. 5,710,178. 4-phenyl butyrate or its derivatives may be obtained from commercial sources, or prepared by total synthesis or semi-synthesis.

In certain embodiments, the chemical chaperone according to the description is a derivative of TUDCA useful in the present description is of the formula: 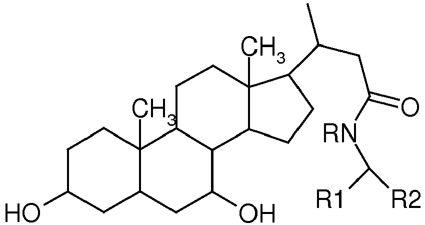 wherein:
R is -H or C1-C4 alkyl;
R1 is -CH2-SO3R3 and R2 is -H; or R1 is -COOH and R2 is -CH2-CH2-CONH2, - CH2-CONH2, -CH2-CH2-SCH3 or -CH2-S-CH2-COOH; and
R3 is -H or a basic amino acid; or a pharmaceutically acceptable salt thereof.

In certain embodiments, R is H. In other embodiments, R is methyl, ethyl, n-propyl, iso-propyl, n-butyl, iso-butyl, or tert-butyl, preferably, methyl. In certain embodiments, R1 or R2 is hydrogen. In certain embodiments, R1 is -CH2-SO3R3 and R2 is -H. In other embodiments, R1 is -COOH and R2 is -CH2-CH2-CONH2, -CH2-CONH2, -CH2-CH2-SCH3 or -CH2-S-CH2-COOH. In certain embodiments, R3 is hydrogen. In certain embodiments, R3 is lysine, arginine, ornithine, or histidine. Derivatives of TUDCA and ursodeoxycholic acid may be obtained from commercial sources, prepared from total synthesis, or obtained from a semi-synthesis. In certain embodiments, the derivative is prepared via semi-synthesis, for example, as described in U.S. Pat. Nos. 5,550,421 and 4,865,765.

The chemical chaperone according to the description may also be a derivative of trim ethylamine N-oxide that has the formula: 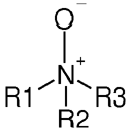 wherein
R1, R2, and R3 are independently hydrogen, halogen, or lower C1-C6 alkyl; or
a pharmaceutically-acceptable salt thereof; or a mixture thereof. In certain embodiments, R1, R2, and R3 are the same. In other embodiments, at least one of R1, R2, and R3 is different. In yet other embodiments, all of R1, R2, and R3 are different. In certain embodiments, R1, R2, and R3 are independently hydrogen or lower C1-C6 alkyl. In yet other embodiments, R1, R2, and R3 are independently lower C1-C6 alkyl. In still other embodiments, R1, R2, and R3 are independently methyl, ethyl, or propyl. In certain embodiments, R1, R2, and R3 are ethyl. Derivatives of TMAO may be obtained from commercial sources, or prepared by total synthesis or semi-synthesis.

Typically, the agent according to the description is a pharmacological chaperone. As used herein, the terms "pharmacological chaperone" refer to a molecule, such as a small molecule, protein, peptide, nucleic acid, or carbohydrate, that enters in cells and specifically binds to a protein and has one or more of the following effects: (i) enhancing the formation of a stable molecular conformation of the protein; (ii) serving as a molecular scaffolding (iii) inducing trafficking of the protein from the ER to another cellular location, preferably a native cellular location, i.e., preventing ER-associated degradation of the protein; (iv) preventing aggregation of misfolded proteins; and/or (v) restoring or enhancing at least partial wild-type function and/or activity to the protein.

In another embodiment the pharmacological chaperone according to the description is isofagomine (IFG; (3R,4R,5R)-5-(hydroxymethyl)-3,4-piperidinediol). IFG has a molecular formula of C 6 H 13 NO3 and a molecular weight of 147.17. This compound is further described in U.S. Pat. Nos. 5,844,102 to Sierks et al., and 5,863,903, to Lundgren et al. N-alkyl IFG derivatives are described in U.S. Pat. No. 6,046,214.

In another embodiment the pharmacological chaperone according to the description is selected from the group consisting of hydroxypiperidine derivatives, which are described in pending PCT publications WO 2005/046611 and WO 2005/046612, and in U.S. patent application Ser. No. 10/988,428, filed Nov. 12, 2004. other examples include glucoimidazole and polyhydroxycyclohexenyl amine derivatives which are described in U.S. patent application Ser. No. 10/988,427 filed on Nov. 12, 2004.

Still other pharmacological chaperones are described in U.S. Pat. No. 6,599,919 to Fan et al., and include calystegine A 3 , calystegine A 5 , calystegine B 1 , calystegine B 2 , calystegine B 3 , calystegine B 4 , calystegine C 1 , N-methyl-calystegine B 2 , DMDP, DAB, castanospermine, N-butyl-isofagomine, N-(3-cyclohexylpropyl)-isofagomine, N-(3-phenylpropyl)-isofagomine, and N-[(2E,6Z,10Z)-3,7,11-trimethyldodecatrienyl]-isofagomine.

In another one, non-limiting embodiment, the pharmacological chaperone may be an inhibitor of glucosidase. Actually, for the quality control of glycoprotein folding, glucosidases are of eminent importance (Roth J. et al. Histochem Cell Biol (2008) 129:163-177; Marcus NY. Et al. the Journal of Biological Chemistry, vol 275 No 3 (2000) 1987-1992). In another embodiment, the pharmacological chaperone may a N-alkyl derivative of 1,5-dideoxy-1,5-imino-D-glucitol. 1,5-Dideoxy-1,5-imino-D-glucitol (or 1-deoxynojirimycin or DNJ) and its N-alkyl derivatives are known inhibitors of the N-linked oligosaccharide processing enzymes, .alpha.-glucosidase I and II. Saunier et al., J. Biol. Chem. 257, 14155-14161 (1982); Elbein, Ann. Rev. Biochem. 56, 497-534 (1987). In a particular embodiment, said alkyl group contains from 2-8 carbon atoms and preferably from 4-6 carbon atoms. In another particular embodiment, the alkyl group is butyl or hexyl. Finally, in another embodiment, the N-alkyl derivative of 1,5-dideoxy-1,5-imino-D-glucitol is 1,5-(butylimino)-1,5-dideoxy-D-glucitol which is also known as miglustast (Zavesca™). Other examples of inhibitors of glucosidase include polyhydroxybenzophenones as described in Hu et al. (Arch Pharme Chem Life Sci 2010 000 1-7).

In another embodiment, the pharmacological chaperone according to the description is an inhibitor of glucosylceramide synthase.

For example said inhibitor of glucosylceramide synthase may be eliglustat tartrate as described in Cox TM. Curr Opin Investing Drugs 2010 Oct;11(10):1169-81 or peterschmitt MJ. Et al. Clin Pharmacol. 2010 Oct 25. Other examples also include D-threo-1-phenyl-2-decanoylamino-3-morpholino-1-propanol (PDMP) and derivatives thereof as described in Abe A. et al. Curr Drug Metab. 2001 Sep;2(3):331-8 such as D-threo-3', 4'-ethylenedioxy-1-phenyl-2-palmitoylamino-3- pyrrolidino-1-propanol and D-threo-4'-hydroxy-1-phenyl-2-palmitoylamino-3- pyrrolidino-1-propanol. Other examples include piperidine derivatives as described in WO02/055498 and 3,4,5-piperidinetriol, 2-(hydroxymethyl)-1-[(4-(pentyloxy)phenyl)methyl]-, (2S,3S,4R,5S) such as decribed in US2007/0259918.

The agents for use according to the invention may be administered in the form of a pharmaceutical composition, as defined below. The agents of the invention may be combined with pharmaceutically acceptable excipients, and optionally sustained-release matrices, such as biodegradable polymers, to form therapeutic compositions.

Accordingly, a further aspect of the description relates to a pharmaceutical composition comprising an agent useful according to the invention for use in a method for treating patients with Darier disease.

The term "Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present description for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The agent of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The agent of the invention may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds of the invention formulated for parenteral administration, such as intravenous or intramuscular injection, other pharmaceutically acceptable forms include, e.g. tablets or other solids for oral administration ; liposomal formulations ; time release capsules ; and any other form currently used.

In a particular embodiment, it may be desirable to administer the agent of the invention in admixture with a topical pharmaceutically acceptable carrier. The topical pharmaceutically acceptable carrier is any substantially nontoxic carrier conventionally usable for topical administration of pharmaceuticals in which the agent of the invention will remain stable and bioavailable when applied directly to skin surfaces. For example, carriers such as those known in the art effective for penetrating the keratin layer of the skin into the stratum comeum may be useful in delivering the agent of the invention to the area of interest. Such carriers include liposomes. agent of the invention can be dispersed or emulsified in a medium in a conventional manner to form a liquid preparation or mixed with a semi-solid (gel) or solid carrier to form a paste, powder, ointment, cream, lotion or the like.

Suitable topical pharmaceutically acceptable carriers include water, buffered saline, petroleum jelly (vaseline), petrolatum, mineral oil, vegetable oil, animal oil, organic and inorganic waxes, such as microcrystalline, paraffin and ozocerite wax, natural polymers, such as xanthanes, gelatin, cellulose, collagen, starch, or gum arabic, synthetic polymers, alcohols, polyols, and the like. The carrier can be a water miscible carrier composition. Such water miscible, topical pharmaceutically acceptable carrier composition can include those made with one or more appropriate ingredients outset of therapy.

Because dermatologic conditions to be treated may be visible, the topical carrier can also be a topical acceptable carrier. The topical acceptable carrier will be any substantially non-toxic carrier conventionally usable for topical administration in which agent of the invention will remain stable and bioavailable when applied directly to the skin surface. Suitable cosmetically acceptable carriers are known to those of skill in the art and include, but are not limited to, cosmetically acceptable liquids, creams, oils, lotions, ointments, gels, or solids, such as conventional cosmetic night creams, foundation creams, suntan lotions, sunscreens, hand lotions, make-up and make-up bases, masks and the like. Topical acceptable carriers may be similar or identical in nature to the above described topical pharmaceutically acceptable carriers.

It may be desirable to have a delivery system that controls the release of agent of the invention to the skin and adheres to or maintains itself on the skin for an extended period of time to increase the contact time of the agent of the invention on the skin. Sustained or delayed release of agent of the invention provides a more efficient administration resulting in less frequent and/or decreased dosage of agent of the invention and better patient compliance. Examples of suitable carriers for sustained or delayed release in a moist environment include gelatin, gum arabic, xanthane polymers. Pharmaceutical carriers capable of releasing the agent of the invention when exposed to any oily, fatty, waxy, or moist environment on the area being treated, include thermoplastic or flexible thermoset resin or elastomer including thermoplastic resins such as polyvinyl halides, polyvinyl esters, polyvinylidene halides and halogenated polyolefins, elastomers such as brasiliensis, polydienes, and halogenated natural and synthetic rubbers, and flexible thermoset resins such as polyurethanes, epoxy resins and the like. Controlled delivery systems are described, for example, in U.S. Pat. No. 5,427,778 which provides gel formulations and viscous solutions for delivery of the agent of the invention to a skin site. Gels have the advantages of having a high water content to keep the skin moist, the ability to absorb skin exudate, easy application and easy removal by washing. Preferably, the sustained or delayed release carrier is a gel, liposome, microsponge or microsphere. The agent of the invention can also be administered in combination with other pharmaceutically effective agents including, but not limited to, antibiotics, other skin healing agents, and antioxidants.

A further object of the invention relates to a method for treating Darier disease comprising administering a subject in need thereof with a therapeutically effective amount of an agent for use according to the invention.

By a "therapeutically effective amount" is meant a sufficient amount of the agent to treat Darier disease at a reasonable benefit/risk ratio applicable to any medical treatment.

It will be understood that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the agent for the symptomatic adjustment of the dosage to the subject to be treated. A medicament typically contains from about 0.01 mg to about 500 mg of the agent, preferably from 1 mg to about 100 mg of the agent. An effective amount of the drug is ordinarily supplied at a dosage level from 0.0002 mg/kg to about 20 mg/kg of body weight per day, especially from about 0.001 mg/kg to 7 mg/kg of body weight per day.

The invention will be further illustrated by the following figures and examples. However, these examples and figures should not be interpreted in any way as limiting the scope of the present invention.

### EXAMPLE 1: SERCA2 DYSFUNCTION IN DARIER DISEASE CAUSES CONSTITUTIVE ER STRESS AND IMMATURE ADHERENS JUNCTIONS : REVERSION BY CHAPERONE MOLECULES

### Material & Methods

**DD patients and molecular diagnostic**. Biopsies from Darier patients were taken from the unaffected (perilesional) skin. Control biopsies of normal skin were obtained from five age- and sex-matched controls. The five Darier patients (D1 to D5) were screened for mutations in the ATP2A2 gene by sequencing the 21 exons and flanking splice sites of ATP2A2. In the following study, we have compared keratinocytes from these five DD patients (D1 to D5) to normal keratinocytes from six healthy controls (NK).

**Cell Culture.** Keratinocytes were grown in 0.06 mM CaCl₂ EpiLife medium (Invitrogen) to 60-80% confluence and then incubated in 1.2 mM CaCl₂ EpiLife medium for 4 hours to induce formation of cell-cell contacts. Experiments were performed at comparable passages between NK and DK.

**Treatments with drugs.** For ER stress induction, keratinocytes grown in high Ca²⁺ (1.2 mM) medium were incubated with thapsigargin (TG, Sigma) at 1µM for different time periods. For inhibition of SERCA pump in NK, keratinocytes were pretreated with 100nM of TG for 30 min in low Ca²⁺ (0.06 mM) medium. Medium was removed, and cells were incubated in high Ca²⁺ (1.2 mM) medium for an additional 4 hours to induce the formation of cell-cell contacts. For chaperone molecules treatment, keratinocytes were pre-treated with sodium 4-phenylbutyrate (PBA, 5mM, Calbiochem), Miglustat and its inactive imino-sugar analogue (NB-DGJ) (500)µM, a kind gift from F. Becq) for 24h before changing the medium for high Ca²⁺ (1.2 mM) medium.

**Immunofluorescence.** Keratinocytes were cultured on coverslips, fixed with 4% paraformaldehyde for 30 minutes at room temperature, and permeabilized with 0.2% TritonX-100 in PBS for 10 minutes. After blocking with 1% fetal bovine serum in PBS, cells were incubated with primary antibodies at 4°C overnight. Subsequently, cells were incubated with the appropriate Alexa-conjugated secondary antibody at room temperature for 1 hour. For actin staining, cells were incubated with phalloidin-TRITC at room temperature for 1 hour. Lastly, coverslips were mounted on glass slides using Vectashield Hard set (Vector laboratories) and examined using a Zeiss LSM 710 confocal microscope with a 40 Plan-Apochromat objective (1.3 oil) or a 63 Plan-Apochromat objective (1.4 oil), together with a 2x zoom.

**Quantification of intracellular compartment volumes and co-localization.** For volume quantifications, all Z-series reconstructions were imaged with a pinhole of 1 Airy Unit with an optimal Z-step. LSM Z-series files were opened in the Imaris 7.1.1 (Bitplane Inc.) software as volumes and then isosurfaced using a 0.2 µm Gaussian filter. Isosurfacing of each intracellular compartment was visually inspected to ensure that it followed closely to the underlying original three-dimensional image and did not incorporate voxels outside of the observable intracellular compartment region. Each isosurfaced was selected and the volume statistic recorded. Quantification of the co-localization signals was performed using the Metamorph (Molecular Devices) software. In a single optical section, the surface of the red signal (E-cadherin) that merges with a green signal (Calnexin) was calculated.

**Western-Blot.** Keratinocytes were lysed in buffer containing 50mM Tris (pH8), 120mM NaCl, 0.5% NP-40, Complete™ protease inhibitors cocktail (Roche) and phosphatase inhibitor cocktail 2 (Sigma) for 30 minutes on ice and pelleted by centrifugation. The supernatant was collected for the quantification of protein amount by the Bradford assay. Equal amounts of soluble protein were added to Laemmli buffer (62.5 mM Tris HCl, pH 6.8, 5% ®-mercaptoethanol, 2% SDS, 10% glycerol, and 0.002% bromophenol blue). Samples were separated by SDS-PAGE and transferred onto Hybond-C extra membrane (GE Healthcare). β-actin immunodetection was used as a loading control. Blots were developed by chemiluminescence (ECL Advance, GE Healthcare or SuperSignal West Dura, Pierce).

**RT-PCR.** RNA was isolated from keratinocytes using TRIzol (Invitrogen) and reverse transcribed using hexamer primers and Moloney murine leukemia virus RT. PCR amplification was performed.

**Transduction of keratinocytes with retroviral vectors.** For transduction of keratinocytes, conditioned medium containing recombinant retrovirus was supplemented with 4 µg/ml of polybrene. The medium was changed 16 h after the addition of the virus, and the cells were allowed to express the protein of interest for 72 h before immunofluorescence experiments. Typical infection efficiency was 90%, as assessed using the reporter gene GFP.

**Statistical analysis**. Student's unpaired two-tailed t-test was used for statistical analyses. P values of 0.05 or less were considered significant. Asterisks represent statistical significance versus the appropriate control in each case * P < 0.05, ** P < 0.01 and *** P < 0.001.

**Study approval**. This study was conducted in compliance with the ethical principles of the Declaration of Helsinki and was approved by the medical ethical committee of the Purpan Hospital in Toulouse. All patients gave informed consent.

### Results

### Darier keratinocytes display a constitutive ER stress response

Decreased [Ca²⁺]_{ER}, a characteristic feature of DK(3-6), triggers ER stress in many cells(18, 19). We show that loss of one *ATP2A2* allele in DK is sufficient to increase eIF2α and IRE1 phosphorylation, i.e. in the absence of exogenous stressor, as compared to normal keratinocytes (NK). This result discloses a constitutive ER stress in DK. After exposure to thapsigargin (TG), a highly specific inhibitor of SERCA pumps(20), the eIF2α and IRE1 phosphorylation is increased in DK to an extent which is higher than in NK (2- to 3-fold in DK and 1.5-fold in NK for phospho-eIF2α). Moreover, the spliced, active form of XBP1 mRNA, the target of phosphorylated-RNAse IRE1, was increased in DK compared to NK, after TG treatment, consistent with up-activation of IRE1 observed in DK. eIF2α and IRE1 phosphorylation and XBP1 splicing were enhanced in DK from the five patients studied (D1 to D5), establishing that DK are more susceptible to ER stress response induced by stressors than NK.

Immunostaining for specific markers calnexin, ERGIC-53, GM130 and TGN46 showed respectively, the ER, ERGIC (ER Golgi intermediate compartment), *cis*-Golgi and *trans*-Golgi compartments to be strictly localized to a perinuclear zone in NK. In contrast, calnexin staining is diffuse all over the cytoplasm in DK in basal conditions. Specific staining for each post-ER compartment shows spots dispersed throughout the cytoplasm, indicating their fragmentation. Quantitative measurements showed a significant increase in volumes of each compartment. All these morphologic changes have been described as induced by ER stress in different cell types(18, 21).

Of note, the number of confluent cells per field was always smaller for DK than for NK, suggesting that DK spread more than NK, at comparable passage numbers.

Together, these data show that DK display different hallmarks of ER stress response under stressor-free conditions.

### Ca²⁺-induced adherens junctions formation is impaired in DK

The shift of 0.06mM to 1.2mM of extracellular calcium concentration induces cell-to-cell contacts and the formation of adhesion complexes. The presence of a constitutive ER stress in DK is likely to alter important ER functions, such as folding of secretory and (trans)membrane proteins (reviewed in(18)). Loss of cell-to-cell adhesion (acantholysis) being the main histological feature in Darier skin lesions, we first investigated the localization of E-cadherin, the transmembrane protein of AJ whose addressing to the plasma membrane is one of the earliest cellular responses to Ca²⁺ increase leading to cellular adhesion. Under low-calcium conditions (0.06mM), E-cadherin was diffusely distributed in the cytoplasm in NK and DK. After 4 hours in high-calcium medium (1.2mM) to induce cell-cell adhesion, E-cadherin molecules relocated to the plasma membrane in NK, with a thin staining around the cellular periphery, forming a continuous belt along cell-cell boundaries. E-cadherin staining in DK was dramatically different compared to NK: punctuated clusters were detected at the cell-cell boundaries, with abnormal organization in strands arranged perpendicularly or at different angles to the cell edge. Contrary to NK, the intercellular space seen between adjacent DK evidenced loss of adherence between these cells.

The localization of different catenins involved in the AJ complexes was also modified in DK. Following incubation in high-calcium medium (1.2mM), catenin proteins moved to the plasma membrane in NK and formed a thin linear continuous belt along the cell-cell boundaries, while in DK all catenin staining patterns displayed a punctuated pattern underneath the plasma membrane. Under low-calcium conditions (0.06mM), β-, α- and p120-catenins were distributed diffusely in the cytosol in NK and DK.

During AJ formation, sequential binding of catenins to E-cadherin induces actin polymerization as observed in NK, in which F-actin appeared as thin cortical bundles underneath and parallel to the plasma membrane. By contrast, F-actin filaments formed thick and disorganized bundles in DK. These findings provide evidence for incorrect localization of AJ components, revealing abnormal formation of these early adhesion structures in DK with an effect on F-actin filaments organisation.

The expression levels of each AJ protein were similar between NK and DK as revealed by immunoblotting analyses suggesting that abnormal AJ formation in DK is not a consequence of reduced expression level. Of note, after 4 hours of exposure to 1.2mM of Ca²⁺ (time to allow for AJ formation), eIF2α phosphorylation was increased in DK compared to NK. Thus, the decrease of Ca²⁺-induced AJ formation correlates with increased ER stress response in DK.

### Thapsigargin inhibits the formation of adherens junctions in NK

To test whether defective AJ assembly in DK is a direct consequence of SERCA2 loss of function, we treated NK with TG to specifically inhibit SERCA pumps. TG induced an enlargement of the ER compartment characterized by a two-fold increase of ER volume in TG-treated NK compared to untreated NK. Of note, the number of confluent cells per microscopy field was always smaller in TG-treated NK than in untreated NK, as observed between DK and NK. Immunofluorescence analysis of AJ in TG-pretreated NK cultured in 1.2mM Ca²⁺ revealed AJ abnormalities similar to those in DK: E-cadherin and catenins were organized into strands arranged at an angle with the cell edge, the staining was punctuated at the cell-cell boundaries, and the F-actin network appeared disorganized in the cytosol. These results demonstrate that ER spreading and defective AJ formation observed in DK are a direct consequence of SERCA2 loss of function.

### Decreased adherens junction formation in DK is due to a selective defect of E-cadherin trafficking to the plasma membrane

Newly synthesized E-cadherin requires binding of β-catenin to facilitate its transport out of the ER to the plasma membrane(22). To distinguish between a selective defect in E-cadherin trafficking and impaired ER exit caused by lack of β-catenin recruitment to the plasma membrane, we expressed an E-cadherin mutant targeted to the plasma membrane independently of β-catenin binding(23). NK and DK were transduced with viral particles (MSCV-EcadM-GFP) encoding this E-cadherin mutant, under low-calcium (0.06mM) conditions to prevent plasma membrane localization of endogenous E-cadherin. We observed a thin, pericellular staining of E-cadherin mutant at the plasma membrane, in addition to a cytosolic fraction, as previously reported in keratinocyte lines(23). Interestingly, β-catenin relocalized to the plasma membrane in DK as observed in NK. NK and DK transduced with a control MSCV-IRES-GFP vector, expressing GFP alone, showed neither E-cadherin staining, nor β-catenin re-localization to the plasma membrane under low-calcium conditions. These results show that endogenous β-catenin in DK is able to localize to the plasma membrane in linear staining, demonstrating that impaired AJ formation in DK is due to a selective defect in E-cadherin trafficking to the plasma membrane.

### E-cadherin is retained in the ER in DK and in TG-treated NK

Using an antibody against the intracellular region of E-cadherin, we confirmed the thick and punctuated staining of E-cadherin at the plasma membrane in DK and TG-treated NK compared to NK. In addition, we observed intracellular retention of E-cadherin in DK and in TG-treated NK as a perinuclear signal that was absent from NK, indicating impaired E-cadherin trafficking to the plasma membrane.

To identify the compartment in which E-cadherin is retained in DK, we performed co-immunofluorescence for E-cadherin and calnexin, a marker of the ER. Intracellular E-cadherin co-localized with calnexin in DK and TG-treated NK. Quantification of E-cadherin intracellular surface overlapping with the calnexin positive compartment demonstrated significant E-cadherin retention in the ER in DK from the five patients studied and in TG-treated NK.

### Treatment of DK with chaperone molecules restores adherens junction formation

Chemical chaperones such as 4-phenylbutyric acid (PBA) alleviate ER stress and restore protein folding and stability in disease models(24, 25). PBA is, therefore, an elegant tool to examine the functional consequences of ER stress on AJ formation in DK. PBA-treatment of DK decreased TG-induced eIF2α phosphorylation and restored perinuclear condensation of the ER, similar to NK. Moreover, PBA-treated DK acquired a pronounced polygonal shape (characteristic of cell-to-cell adhesion) whose features are: a linear, sharp and more intense pericellular staining of E-cadherin and catenins; cortical bundles of F-actin parallel and closer to the cell membrane. Altogether these data show that PBA decreases ER stress and corrects AJ formation in DK.

We also tested the pharmacologic chaperone Miglustat (N-butyldeoxynojirimycin, Zavesca^{®}). This molecule has been proposed for clinical use in cystic fibrosis due to its capacity to correct defective trafficking of ΔF508-CFTR(26). Neither TG-induced eIF2α phosphorylation nor ER expansion were reduced in Miglustat-treated DK. However, Miglustat induced a narrow, intense, continuous pericellular staining of all AJ components at the plasma membrane of DK, as well as polymerization of the F-actin network. Its inactive analogue (NB-DGJ) had not effect on E-cadherin localization. These results were obtained with 500µM Miglustat, a concentration at which all cells present a polygonal phenotype. At lower concentration (100)µM), Miglustat induced increased plasma membrane localization of E-cadherin only in a limited number of cells. No toxicity of chaperone molecules toward keratinocytes was observed during these protocols.

The effects of PBA and Miglustat were observed reproducibly on keratinocytes from the five DD patients studied. Of note, neither PBA nor Miglustat increased E-cadherin and SERCA2 expression in DK. These results demonstrate full rescue of AJ formation by chaperon molecules in DK.

### Discussion:

This study demonstrates for the first time that DK display a constitutive ER stress response leading to defective AJ formation, and that treatment with chaperone molecules restores mature AJ formation.

The [Ca²⁺]_{ER} plays a key role in ER-related functions(27), ER remodeling(28), post-ER compartment architecture(21), and ER-stress response(18, 19). Here, we show that, SERCA2 dysfunction, through lowering [Ca²⁺]_{ER}(5, 6), induced a constitutive ER stress. In DK, the ER is diffuse throughout the cytoplasm, all ER-post-secretory compartments are disorganized and fragmented, and the cell size is increased compared to NK, as previously described in other ER-stressed cells(29). ER expansion and cellular enlargement were recapitulated in NK treated by a SERCA2 inhibitor, demonstrating that these morphological changes are a direct consequence of the loss of SERCA2 function in DK.

Darier disease onset is usually around puberty and runs a chronic relapsing course, with exacerbations by stressors such as UVB irradiation, heat, friction and infections. Remarkably, under basal conditions, eIF2α and IRE1 phosphorylation are enhanced in DK, demonstrating that loss of one *ATP2A2* allele is sufficient to induce an ER stress response in stressor-free conditions. The triggering factors of clinical manifestations could act by exacerbating the constitutive ER stress of DK, as we observed with TG. Consistent with this notion, UVB irradiation is known to induce an ER stress response(30). The presence of rounded keratinocytes (corps ronds), which are characteristic of DD and thought to correspond to apoptotic cells, could result from an uncontrolled ER stress response triggered by external factors, leading to apoptosis.

Skin sections from Darier patients are histologically characterized by acantholysis leading to suprabasal clefts. AJ formation, one of the earliest events following the increase in extracellular calcium concentration(11), is known to proceed through different stages, immature AJ being one of the earliest⁴². In DK, we observed two rows of co-localized E-cadherin/catenin molecules as puncta at the edges of cell-cell contacts and actin filaments approached initial cell contacts perpendicularly(31-34). These are features of the first step of AJ formation and typical of immature AJ. By contrast, under the same conditions, NK displayed linear staining of E-cadherin and catenins at the plasma membrane, which is characteristic of mature AJ. We concluded that DK form immature AJ, which predispose DD epidermis to defective intercellular adhesion when exposed to triggering factors. TG-treated NK developed the same immature AJ, demonstrating that loss of SERCA2 function is the cause of defect in AJ formation.

We provide evidence that the formation of immature AJ in DK is due to defective trafficking of E-cadherin to the plasma membrane: 1) expression levels of AJ component proteins are comparable between DK and NK; 2) activated ER stress response in DK and in TG-treated NK leads to E-cadherin retention in the ER; and 3) expressing recombinant E-cadherin that constitutively localizes to the cell membrane in low-Ca²⁺ conditions is sufficient to relocate endogenous β-catenin to the cell membrane.

The current treatment for DD is not satisfactory, and relies mainly on the use of topical or oral retinoids (acitretin or isotretinoin) which are often not well tolerated and not fully effective(35). PBA has been shown to act as a chemical chaperone in several protein misfolding diseases, allowing for folding of the mutated protein and its proper localization in the cell by relieving ER stress (24-26, 36). We demonstrate here that PBA attenuates the ER stress response in DK, as evidenced by decreasing eIF2α phosphorylation and ER volume, and restores trafficking of AJ proteins to the plasma membrane to form mature AJ. These data establish a causal role for ER stress in immature AJ formation. In addition, Miglustat, a pharmacological chaperone known to allow proper localization of mutated CFTR(26), restores trafficking of AJ molecules to the plasma membrane and the formation of mature AJ in DK. We demonstrate for the first time that these chaperone molecules are able to restore proper localization of a non-mutated protein retained in the ER as a result of an ER stress induced by Ca²⁺-depletion.

The precise mechanisms of action of these molecules are unclear. Although PBA has been described as a transcriptional regulator(37), neither E-cadherin nor SERCA2 expression are modified in PBA-treated DK. In contrast, decreased ER stress response and re-localization of AJ molecules to the plasma membrane in PBA-treated DK suggest that PBA could act via its chemical chaperone activity by preventing aggregation of misfolded mutant proteins and alleviating ER stress, as previously reported(38).

In cystic fibrosis, Miglustat prevents the interaction between ΔF508-CFTR and calnexin, a Ca²⁺-dependent ER chaperone protein, enabling a functional ΔF508-CFTR to reach the plasma membrane(26). In DK, Miglustat may release E-cadherin from calnexin, thus allowing its trafficking from the ER to the plasma membrane.

In conclusion, these results establish that a constitutive ER stress causes immature AJ formation in DK. ER stress would be a general mechanism accounting for defective AJ formation, but also impaired desmoplakin trafficking as we previously described(10), underlying loss of cell-to-cell adhesion in DD epidermis. This work highlights the crucial role of Ca²⁺ ER concentration in the physiopathology of DD, and allows classifying DD as an ER stress-related disease involving protein misfolding. Moreover, because chaperone molecules have already been successfully used in animal and/or clinical trials(24, 25, 39-41), our study provides an important proof of principle supporting a future clinical application in Darier patients.

### REFERENCES:

Throughout this application, various references describe the state of the art to which this invention pertains. The disclosures of these references are hereby incorporated by reference into the present disclosure.
1. Burge, S., and Hovnanian, A. 2011. Acantholytic Disorders of the Skin. Chapter 51, in Dermatology in General Medecine 2011, 8th edition, Fitzpatrick's
2. Sakuntabhai, A., Ruiz-Perez, V., Carter, S., Jacobsen, N., Burge, S., Monk, S., Smith, M., Munro, C.S., O'Donovan, M., Craddock, N., et al. 1999. Mutations in ATP2A2, encoding a Ca2+ pump, cause Darier disease. Nat Genet 21:271-277.
3. Dode, L., Andersen, J.P., Leslie, N., Dhitavat, J., Vilsen, B., and Hovnanian, A. 2003. Dissection of the functional differences between sarco(endo)plasmic reticulum Ca2+-ATPase (SERCA) 1 and 2 isoforms and characterization of Darier disease (SERCA2) mutants by steady-state and transient kinetic analyses. J Biol Chem 278:47877-47889.
4. Miyauchi, Y., Daiho, T., Yamasaki, K., Takahashi, H., Ishida-Yamamoto, A., Danko, S., Suzuki, H., and Iizuka, H. 2006. Comprehensive analysis of expression and function of 51 sarco(endo)plasmic reticulum Ca2+-ATPase mutants associated with Darier disease. J Biol Chem 281:22882-22895.
5. Foggia, L., Aronchik, I., Aberg, K., Brown, B., Hovnanian, A., and Mauro, T.M. 2006. Activity of the hSPCA1 Golgi Ca2+ pump is essential for Ca2+-mediated Ca2+ response and cell viability in Darier disease. J Cell Sci 119:671-679.
6. Pani, B., Cornatzer, E., Cornatzer, W., Shin, D.M., Pittelkow, M.R., Hovnanian, A., Ambudkar, I.S., and Singh, B.B. 2006. Up-regulation of transient receptor potential canonical 1 (TRPC1) following sarco(endo)plasmic reticulum Ca2+ ATPase 2 gene silencing promotes cell survival: a potential role for TRPC1 in Darier's disease. Mol Biol Cell 17:4446-4458.
7. Hovnanian, A. 2004. Darier's disease: from dyskeratosis to endoplasmic reticulum calcium ATPase deficiency. Biochem Biophys Res Commun 322:1237-1244.
8. Savignac, M., Edir, A., Simon, M., and Hovnanian, A. 2011. Darier disease : a disease model of impaired calcium homeostasis in the skin. Biochim Biophys Acta 1813:1111-1117.
9. Liu, L.H., Boivin, G.P., Prasad, V., Periasamy, M., and Shull, G.E. 2001. Squamous cell tumors in mice heterozygous for a null allele of Atp2a2, encoding the sarco(endo)plasmic reticulum Ca2+-ATPase isoform 2 Ca2+ pump. J Biol Chem 276:26737-26740.
10. Dhitavat, J., Cobbold, C., Leslie, N., Burge, S., and Hovnanian, A. 2003. Impaired trafficking of the desmoplakins in cultured Darier's disease keratinocytes. J Invest Dermatol 121:1349-1355.
11. Wheelock, M.J., and Johnson, K.R. 2003. Cadherins as modulators of cellular phenotype. Annu Rev Cell Dev Biol 19:207-235.
12. Getsios, S., Huen, A.C., and Green, K.J. 2004. Working out the strength and flexibility of desmosomes. Nat Rev Mol Cell Biol 5:271-281.
13. Perez-Moreno, M., Jamora, C., and Fuchs, E. 2003. Sticky business: orchestrating cellular signals at adherens junctions. Cell 112:535-548.
14. Pokutta, S., and Weis, W.I. 2007. Structure and mechanism of cadherins and catenins in cell-cell contacts. Annu Rev Cell Dev Biol 23:237-261.
15. Berridge, M.J., Bootman, M.D., and Roderick, H.L. 2003. Calcium signalling: dynamics, homeostasis and remodelling. Nat Rev Mol Cell Biol 4:517-529.
16. Yoshida, H. 2007. ER stress and diseases. Febs J 274:630-658.
17. Schroder, M., and Kaufman, R.J. 2005. ER stress and the unfolded protein response. Mutat Res 569:29-63.
18. Schroder, M., and Kaufman, R.J. 2005. The mammalian unfolded protein response. Annu Rev Biochem 74:739-789.
19. Ashby, M.C., and Tepikin, A.V. 2001. ER calcium and the functions of intracellular organelles. Semin Cell Dev Biol 12:11-17.
20. Thastrup, O., Dawson, A.P., Scharff, O., Foder, B., Cullen, P.J., Drobak, B.K., Bjerrum, P.J., Christensen, S.B., and Hanley, M.R. 1989. Thapsigargin, a novel molecular probe for studying intracellular calcium release and storage. Agents Actions 27:17-23.
21. Amodio, G., Renna, M., Paladino, S., Venturi, C., Tacchetti, C., Moltedo, O., Franceschelli, S., Mallardo, M., Bonatti, S., and Remondelli, P. 2009. Endoplasmic reticulum stress reduces the export from the ER and alters the architecture of post-ER compartments. Int J Biochem Cell Biol 41:2511-2521.
22. Chen, Y.T., Stewart, D.B., and Nelson, W.J. 1999. Coupling assembly of the E-cadherin/beta-catenin complex to efficient endoplasmic reticulum exit and basal-lateral membrane targeting of E-cadherin in polarized MDCK cells. J Cell Biol 144:687-699.
23. Fukumoto, Y., Shintani, Y., Reynolds, A.B., Johnson, K.R., and Wheelock, M.J. 2008. The regulatory or phosphorylation domain of p120 catenin controls E-cadherin dynamics at the plasma membrane. Exp Cell Res 314:52-67.
24. Erbay, E., Babaev, V.R., Mayers, J.R., Makowski, L., Charles, K.N., Snitow, M.E., Fazio, S., Wiest, M.M., Watkins, S.M., Linton, M.F., et al. 2009. Reducing endoplasmic reticulum stress through a macrophage lipid chaperone alleviates atherosclerosis. Nat Med 15:1383-1391.
25. Ozcan, U., Yilmaz, E., Ozcan, L., Furuhashi, M., Vaillancourt, E., Smith, R.O., Gorgun, C.Z., and Hotamisligil, G.S. 2006. Chemical chaperones reduce ER stress and restore glucose homeostasis in a mouse model of type 2 diabetes. Science 313:1137-1140.
26. Norez, C., Noel, S., Wilke, M., Bijvelds, M., Jorna, H., Melin, P., DeJonge, H., and Becq, F. 2006. Rescue of functional delF508-CFTR channels in cystic fibrosis epithelial cells by the alpha-glucosidase inhibitor miglustat. FEBS Lett 580:2081-2086.
27. Federovitch, C.M., Ron, D., and Hampton, R.Y. 2005. The dynamic ER: experimental approaches and current questions. Curr Opin Cell Biol 17:409-414.
28. Sammels, E., Parys, J.B., Missiaen, L., De Smedt, H., and Bultynck, G. 2010. Intracellular Ca2+ storage in health and disease: a dynamic equilibrium. Cell Calcium 47:297-314.
29. Shaffer, A.L., Shapiro-Shelef, M., Iwakoshi, N.N., Lee, A.H., Qian, S.B., Zhao, H., Yu, X., Yang, L., Tan, B.K., Rosenwald, A., et al. 2004. XBP1, downstream of Blimp-1, expands the secretory apparatus and other organelles, and increases protein synthesis in plasma cell differentiation. Immunity 21:81-93.
30. Mera, K., Kawahara, K., Tada, K., Kawai, K., Hashiguchi, T., Maruyama, I., and Kanekura, T. 2010. ER signaling is activated to protect human HaCaT keratinocytes from ER stress induced by environmental doses of UVB. Biochem Biophys Res Commun 397:350-354.
31. Yonemura, S., Itoh, M., Nagafuchi, A., and Tsukita, S. 1995. Cell-to-cell adherens junction formation and actin filament organization: similarities and differences between non-polarized fibroblasts and polarized epithelial cells. J Cell Sci 108 (Pt 1):127-142.
32. Adams, C.L., Chen, Y.T., Smith, S.J., and Nelson, W.J. 1998. Mechanisms of epithelial cell-cell adhesion and cell compaction revealed by high-resolution tracking of E-cadherin-green fluorescent protein. J Cell Biol 142:1105-1119.
33. Adams, C.L., Nelson, W.J., and Smith, S.J. 1996. Quantitative analysis of cadherin-catenin-actin reorganization during development of cell-cell adhesion. J Cell Biol 135:1899-1911.
34. Vasioukhin, V., Bauer, C., Yin, M., and Fuchs, E. 2000. Directed actin polymerization is the driving force for epithelial cell-cell adhesion. Cell 100:209-219.
35. Burge, S. 1999. Management of Darier's disease. Clin Exp Dermatol 24:53-56.
36. Chamcheu, J.C., Pihl-Lundin, I., Mouyobo, C.E., Gester, T., Virtanen, M., Moustakas, A., Navsaria, H., Vahlquist, A., and Torma, H. 2010. Immortalized keratinocytes derived from epidermolytic ichthyosis patients reproduce the disease phenotype: a useful in vitro model for testing new treatments. Br J Dermatol.
37. Dover, G.J., Brusilow, S., and Samid, D. 1992. Increased fetal hemoglobin in patients receiving sodium 4-phenylbutyrate. N Engl J Med 327:569-570.
38. Kubota, K., Niinuma, Y., Kaneko, M., Okuma, Y., Sugai, M., Omura, T., Uesugi, M., Uehara, T., Hosoi, T., and Nomura, Y. 2006. Suppressive effects of 4-phenylbutyrate on the aggregation of Pael receptors and endoplasmic reticulum stress. J Neurochem 97:1259-1268.
39. Cox, T.M., Aerts, J.M., Andria, G., Beck, M., Belmatoug, N., Bembi, B., Chertkoff, R., Vom Dahl, S., Elstein, D., Erikson, A., et al. 2003. The role of the iminosugar N-butyldeoxynojirimycin (miglustat) in the management of type I (non-neuronopathic) Gaucher disease: a position statement. J Inherit Metab Dis 26:513-526.
40. Rubenstein, R.C., and Zeitlin, P.L. 1998. A pilot clinical trial of oral sodium 4-phenylbutyrate (Buphenyl) in deltaF508-homozygous cystic fibrosis patients: partial restoration of nasal epithelial CFTR function. Am J Respir Crit Care Med 157:484-490.
41. Collins, A.F., Pearson, H.A., Giardina, P., McDonagh, K.T., Brusilow, S.W., and Dover, G.J. 1995. Oral sodium phenylbutyrate therapy in homozygous beta thalassemia: a clinical trial. Blood 85:43-49.

## Claims

1. An agent that is able to restore the E-cadherin trafficking in Darier keratinocytes and the formation of adherens junctions for use in treating a patient with Darier disease, wherein said agent is selected from the group consisting of chemical chaperones and pharmacologic chaperones,
the chemical chaperone being 4-phenyl butyric acid (PBA), and
the pharmacological chaperone being 1,5-(butylimino)-1,5-dideoxy-D-glucitol (miglustat).

## Patentansprüche

1. Wirkstoff, der in der Lage ist, den E-Cadherin-Transport in Darier-Keratinocten und die Bildung von Adhärenz-Verbindungen wiederherzustellen, zur Verwendung bei der Behandlung von Patienten mit Morbus Darier, wobei der Wirkstoff aus der Gruppe ausgewählt ist, die aus chemischen Chaperonen und pharmakologischen Chaperonen besteht, wobei das chemische Chaperon eine 4-Phenylbuttersäure (PBA) ist und das pharmakologische Chaperon ein 1,5-(butylimino)-1,5-dideoxy-D-Glucit (Miglustat) ist.

## Revendications

1. Agent qui est capable de restaurer le trafic de l'E-cadhérine dans des kératinocytes de Darier et la formation de jonctions adhérentes, destiné à être utilisé dans le traitement d'un patient atteint de la maladie de Darier, où ledit agent est sélectionné dans le groupe consistant en des chaperons chimiques et des chaperons pharmacologiques,
le chaperon chimique étant l'acide 4-phénylbutyrique (PBA), et
le chaperon pharmacologique étant le 1,5-(butylimino)-1,5-didésoxy-D-glucitol (miglustat).
